Europäisches Patentamt

European Patent Office

Office européen des brevets

(19)

(11) Numéro de publication : **0 143 132 B1**

(12) **FASCICULE DE BREVET EUROPÉEN**

(45) Date de publication du fascicule du brevet :
02.03.88

(51) Int. Cl.⁴ : **A 61 F   5/01**

(21) Numéro de dépôt : **83402235.2**

(22) Date de dépôt : **21.11.83**

(54) **Appareillage externe pour handicapés moteurs d'au moins un membre supérieur.**

(43) Date de publication de la demande :
**05.06.85 Bulletin 85/23**

(45) Mention de la délivrance du brevet :
**02.03.88 Bulletin 88/09**

(84) Etats contractants désignés :
**AT BE CH DE GB IT LI LU NL SE**

(56) Documents cités :
**DE-A- 2 615 209**
**DE-C-   299 882**
**FR-A- 2 340 080**
**FR-E-.   20 530**
**GB-A- 1 150 072**

(73) Titulaire : **Salort, Guy**
**219, rue Raymond Losserand**
**F-75014 Paris (FR)**

(72) Inventeur : **Salort, Guy**
**219, rue Raymond Losserand**
**F-75014 Paris (FR)**

(74) Mandataire : **Peuscet, Jacques**
**Cabinet Peuscet 68, rue d'Hauteville**
**F-75010 Paris (FR)**

EP 0 143 132 B1

## Description

La présente invention a trait à un appareillage externe pour handicapés moteurs d'au moins un membre supérieur. Cet appareillage permet au sujet de mouvoir son membre handicapé en utilisant exclusivement l'énergie provenant du moteur scapulaire.

Le but de la présente invention est de fournir une orthèse autonome, qui réalise une mise en compression des articulations par des moyens neutralisant d'une façon dynamique le poids du membre. On permet, ainsi, l'information et la réactivation des unités motrices par une modulation de la chute, dans le temps et dans l'espace, du membre handicapé ; on améliore et on accélère ainsi la récupération globale du geste volontaire de la préhension.

Jusqu'à ce jour, dans l'état de la technique, on a proposé des orthèses de membre supérieur de trois types : dans le premier type, les différentes parties du membre' sont enserrées dans des coques que l'on manœuvre les unes par rapport aux autres au moyen d'organes moteurs commandés par le sujet, tels que, par exemple, un moteur électrique ou un muscle artificiel gonflable ; dans le deuxième type, on supporte le membre dans une position donnée au moyen d'une carcasse rigide ; dans le troisième type, illustré par exemple par le document DE-A 2 615 209, on supporte le membre par une structure externe rigide articulée en plusieurs points par des axes de façon à permettre une certaine mobilité des différentes parties du membre l'une par rapport à l'autre, mais le sujet ne peut commander les mouvements de son membre que par une action externe, soit avec un moteur externe, soit par une action de son autre membre. Dans tous les cas, le membre est entièrement supporté et le jeu des articulations, quand il existe, est beaucoup plus limité que le jeu articulaire physiologique.

Dans le document DE-A 2 615 209 précité, on a ainsi décrit un appareillage externe pour handicapés moteurs d'un membre supérieur, qui comprend :

a) une structure thoraco-scapulaire constituée d'une épaulière et d'un sous-ensemble d'amarrage disposé autour du torse ;

b) un para-squelette du membre supérieur et de la main.

Dans cet appareillage, on supporte le membre par une structure externe rigide articulée, qui est commandée par un organe moteur penumatique ou hydraulique et comme ci-dessus indiqué, le membre est entièrement supporté et le jeu des articulations est beaucoup plus limité que le jeu articulaire physiologique.

Au contraire, selon l'invention, le membre peut avoir tous les mouvements physiologiques normaux, les articulations pouvant jouer dans tous les sens physiologiquement admissibles et la commande du membre est effectuée par le sujet lui-même, sans l'aide de moteur externe, en mettant en jeu le moteur scapulaire constitué par la structure de l'épaule. Suivant que le sujet, par les mouvements de son épaule, ouvre ou ferme le cône scapulaire constitué au niveau de la liaison de l'omoplate et de la clavicule, il provoque une action sur l'humérus, d'où résulte, par l'appareillage selon l'invention, un ensemble d'actions sur toutes le parties du membre, qui font jouer les articulations dans les limites du jeu physiologique normal.

L'invention concerne donc un appareillage externe pour handicapés moteurs d'au moins un membre supérieur, qui permet au sujet de mouvoir son membre handicapé, et qui, pour chaque membre handicapé, comporte :

une structure thoraco-scapulaire constituée d'une épaulière et d'un sous-ensemble d'amarrage disposé autour du torse,

un para-squelette du membre supérieur et de la main, appareillage caractérisé en ce que :

1.1 l'épaulière est formée d'au moins trois coquilles semi-rigides reliées entre elles de façon souple, la première étant disposée sur l'angle supérieur du pectoral et passant sur l'arête supérieure du trapèze, la seconde étant disposée sur la zone claviculo-trapézoïdale à la pointe de l'épaule, la troisième étant disposée au droit du scapulum, au moins un tendeur élastique étant prévu entre la seconde coquille et chacune des deux autres ;

1.2 le sous-ensemble d'amarrage disposé autour du torse est relié à l'épaulière en au moins trois points d'accrochage répartis de part et d'autre de l'épaule ;

et que le para-squelette du membre supérieur et de la main comprend :

2.1 cinq éléments de liaison avec le membre disposés respectivement au niveau des parties humérales supérieure et inférieure, des parties radio-cubitales supérieure et inférieure et de la main, l'élément de liaison huméral supérieur comprenant la seconde coquille de l'épaulière et un lien périphérique qui entoure le membre, chacun des quatre autres éléments de liaison comportant une coquille semi-rigide réalisée en une ou plusieurs pièces et au moins un lien périphérique qui entoure le membre ;

2.2 des moyens élastiques disposés entre deux des éléments de liaison susmentionnés, qui comprennent :

2.2.1 un levier-ressort fonctionnant en flexion et en torsion fixé entre la seconde coquille de l'épaulière et la coquille de l'élément de liaison huméral inférieur, sensiblement parallèlement à l'humérus le long de la bordure externe du membre,

2.2.2 un second levier-ressort analogue au premier, fixé entre la coquille de l'élément de liaison radio-cubital supérieur et la coquille de la main, sensiblement parallèlement au cubitus le long de la bordure interne du membre,

2.2.3 deux tendeurs élastiques, disposés sur les bordures externe et interne du bras, entre les éléments de liaison huméral inférieur et radio-cubital supérieur,

2.2.4 deux tendeurs élastiques disposés entre l'élément de liaison radio-cubital inférieur et l'élément de liaison de la main.

Dans un mode préféré de réalisation, le sous-ensemble d'amarrage est relié à l'épaulière en trois points d'accrochage, le premier situé sur la zone antérieure de la première coquille de l'épaulière, le second situé sur la zone inférieure de la troisième coquille, et le troisième situé sur la zone supérieure interne de la troisième coquille ; avantageusement, le sous-ensemble d'amarrage est constitué :

d'une ceinture entourant le sujet au niveau de la taille, ladite ceinture pouvant être d'une largeur plus ou moins importante et pouvant constituer un corset, ladite ceinture étant unique si le sujet est handicapé des deux membres supérieurs et porte un appareillage pour chacun des deux membres ;

d'une liaison antérieure constituée d'une transmission thoracique et d'un raccord élastique, ladite transmission ayant une extrémité fixée sur la ceinture au niveau de l'épine iliaque du côté opposé au membre appareillé et ayant son autre extrémité liée par le raccord élastique précité à la première coquille de l'épaulière ;

d'une première liaison élastique latérale tendue entre la ceinture, au niveau de l'épine iliaque du côté du membre appareillé, et la troisième coquille de l'épaulière ;

d'une deuxième liaison élastique latérale tendue, du côté opposé au membre appareillé, entre la troisième coquille de l'épaulière et la liaison antérieure précitée.

Selon une réalisation préférée, la transmission thoracique est un levier-ressort, qui forme un angle d'environ 30° avec l'axe du corps du sujet ; la première liaison élastique latérale se raccorde à la troisième coquille de l'épaulière, au droit de la base de l'omoplate et la deuxième liaison élastique latérale se raccorde à la troisième coquille de l'épaulière, au voisinage de la partie interne de l'épine de l'omoplate.

On peut avantageusement prévoir que la seconde coquille de l'épaulière soit reliée à la troisième coquille de ladite épaulière par un tendeur fixé entre la zone postéro-supérieure de la seconde coquille et la zone centrale de la troisième coquille, un renvoi d'angle obligeant le tendeur à passer au droit de la partie externe de l'épine de l'omoplate ; de même, on peut prévoir que la seconde coquille de l'épaulière soit reliée à la première coquille par un tendeur fixé entre la zone antéro-supérieure de la deuxième coquille et la zone postéro-interne de la première coquille, un renvoi d'angle obligeant le tendeur à rester au droit de la zone claviculaire sur environ la moitié de sa longueur. Les renvois d'angle de tendeurs peuvent être constitués par des poulies.

Les leviers-ressorts de l'appareillage selon l'invention travaillent généralement en flexion et en torsion. Ils sont avantageusement constitués par des lames métalliques flexibles, réalisées notamment en acier à fortes caractéristiques élastiques.

Dans un mode préféré de réalisation, l'élément de liaison huméral inférieur et l'élément de liaison radio-cubital supérieur comportent, chacun, une seule coquille, les deux coquilles ayant leurs bordures adjacentes séparées par un espace articulaire ayant une largeur sensiblement constante comprise entre 5 et 15 mm environ, et étant reliées, au droit dudit espace, par des moyens élastiques disposés de part et d'autre de l'olécrane, la bordure de la coquille humérale inférieure le long dudit espace présentant une forme convexe au droit de l'épicondyle et de l'épitrochlée et une forme concave au droit de l'olécrane, la bordure de la coquille radio-cubitale supérieure présentant la forme complémentaire ; l'élément de liaison huméral inférieur ou supérieur recouvre, avantageusement, environ le tiers inférieur du bras ; la coquille de l'élément de liaison radio-cubital inférieur est constituée de deux demi-coquilles recouvrant respectivement la partie radiale et la partie cubitale du membre, ces deux demi-coquilles étant séparées par un sillon au droit du cubitus, le membre étant introduit dans la coquille par le côté opposé au sillon, les deux demi-coquilles étant serrées autour du membre par deux liens périphériques disposés aux deux extrémités supérieure et inférieure de ladite coquille ; l'élément de liaison radio-cubital inférieur ou supérieur recouvre environ le tiers de l'avant-bras.

Dans une réalisation préférée, l'élément de liaison de la main comporte, en premier lieu, une coquille constituée de trois segments, le premier segment recouvrant l'éminence thénar, le deuxième segment recouvrant les deuxième, troisième et quatrième carpiens et métacarpiens jusqu'à la base des doigts, le troisième segment recouvrant l'éminence hypothénar et, en second lieu, d'une part, un premier lien périphérique, disposé sur les premier et deuxième segments précités et entourant la main à l'exception du pouce au niveau de la partie inférieure des métacarpiens, et, d'autre part, un deuxième lien périphérique entourant le carpe et passant sur les trois segments au voisinage du poignet, ledit deuxième lien périphérique étant relié à l'élément de liaison radio-cubital inférieur par une bande de caoutchouc ; l'élément de liaison de la main est relié à l'élément de liaison radio-cubital inférieur par deux tendeurs, l'un, entre la base du pouce et la zone radiale inférieure et, l'autre, entre la zone cubitale inférieure et la zone palmaire médiane du métacarpe, passant en diagonale au-dessus de la main ; le second levier-ressort de l'appareillage est disposé en regard de l'espace entre le cubitus et le bord interne de l'avant-bras et il a son extrémité inférieure fixée sur le troisième segment de la coquille de l'élément de liaison de la main, un peu en avant du pisiforme.

Dans une première variante de réalisation de l'élément de liaison de la main, un lien porté par le premier segment de la coquille de la main entoure la base du pouce, les autres doigts de la main n'étant pas appareillés. Dans une deuxième variante, chaque doigt de la main est appareillé par un tendeur élastique croisé disposé entre une borne d'appui placée au-dessus du métacarpe et une borne d'appui placée au-dessus de la seconde phalange ; la borne d'appui placée au niveau du métacarpe est portée par le segment de la coquille de la main, qui lui correspond, et la borne d'appui placée au niveau de la seconde phalange est portée par un doigtier, qui entoure l'extrémité du doigt ; chaque borne d'appui comporte, de préférence, un élément triangulaire à orientation réglable susceptible de tourner autour d'un axe sensiblement perpendiculaire à l'os qu'il surmonte.

Pour mieux faire comprendre l'objet de l'invention, on va en décrire maintenant, à titre d'exemple purement illustratif et non limitatif, un mode de réalisation représenté sur le dessin annexé.

Sur ce dessin :

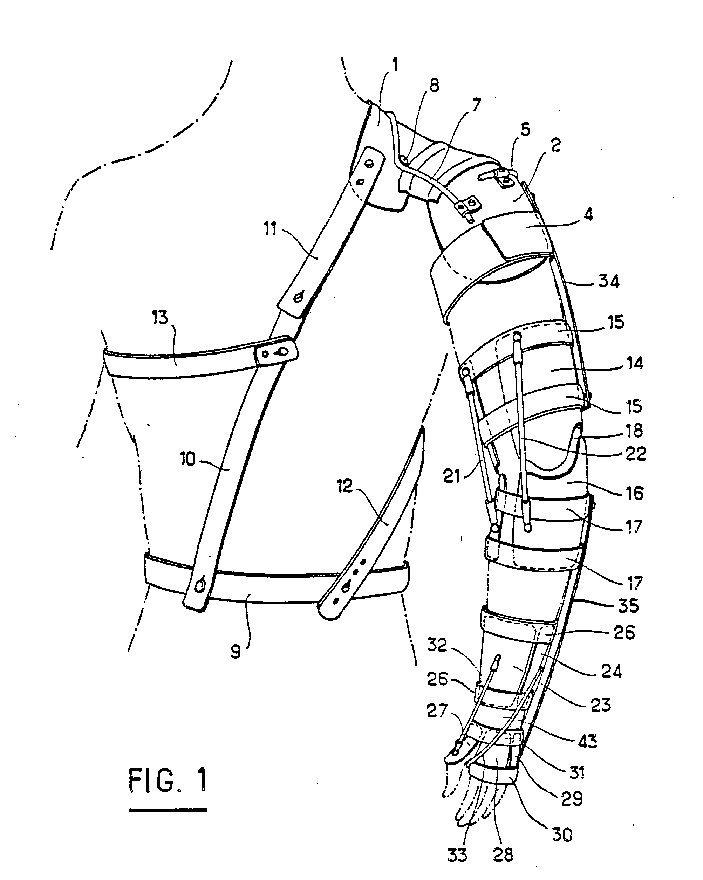

la figure 1 représente une vue schématique antérieure d'un appareillage selon l'invention porté par un sujet handicapé moteur du bras gauche ;

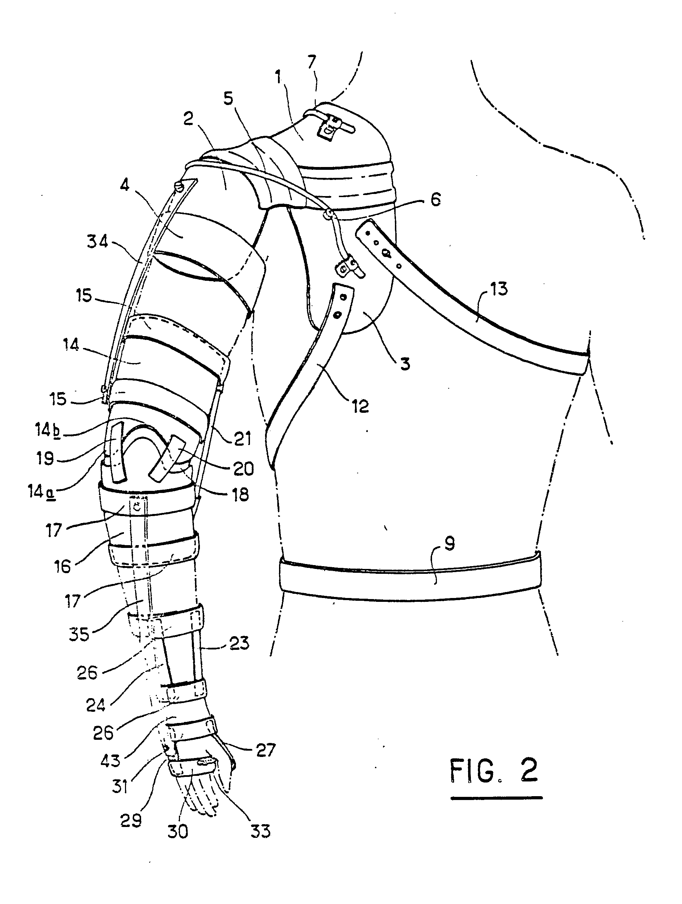

la figure 2 représente une vue schématique postérieure de l'appareillage de la figure 1 ;

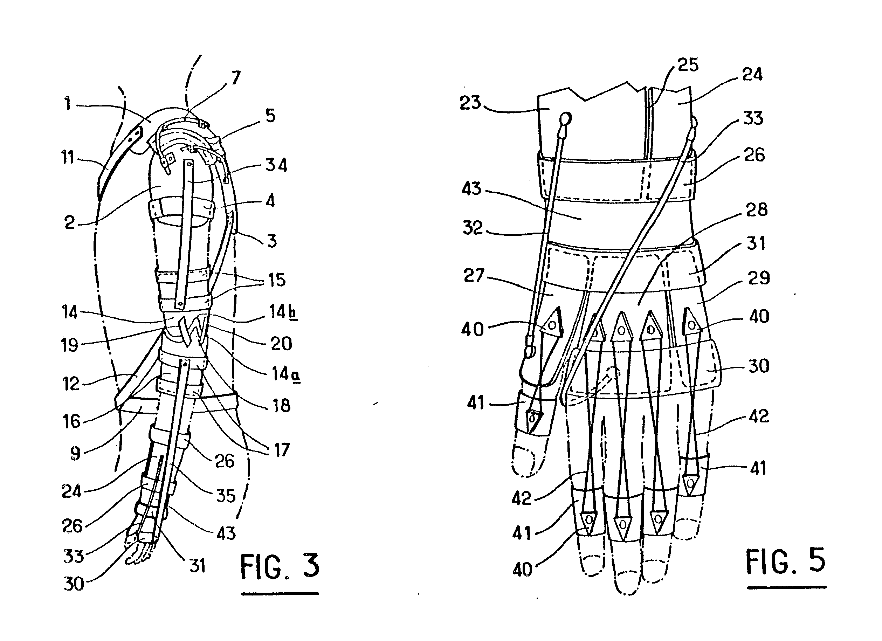

la figure 3 représente une vue latérale externe de l'appareillage des figures 1 et 2 ;

la figure 4 représente, en perspective, une première variante de la coquille de la main, dans laquelle le premier segment est équipé d'un lien pour supporter le pouce ;

la figure 5 représente, vue en plan, une deuxième variante de réalisation de l'élément de liaison de la main, dans laquelle chaque doigt est appareillé avec un tendeur croisé ;

la figure 6 représente schématiquement, en perspective éclatée, la réalisation d'une borne d'appui des tendeurs croisés de la figure 5.

En se référant au dessin, on voit que l'appareillage représenté comporte une structure thoraco-scapulaire constituée d'une épaulière et d'un sous-ensemble d'amarrage.

L'épaulière est formée de trois coquilles semi-rigides en matière plastique moulée. La première coquille 1 est disposée sur l'angle supérieur du pectoral et passe sur l'arête supérieure du trapèze. La seconde coquille 2 est disposée sur la zone claviculo-trapézoïdale à la pointe de l'épaule. La troisième coquille 3 est disposée au droit du scapulum, c'est-à-dire sur l'omoplate. Ces trois coquilles 1, 2, 3 sont reliées entre elles par des bandes de caoutchouc, mais elles sont séparées l'une de l'autre par des espaces suffisants pour permettre leur mouvement relatif. La forme de ces coquilles est adaptée anatomiquement au sujet qui doit les porter.

La coquille 2 de l'épaulière est solidaire d'un lien périphérique 4, qui est destiné à entourer élastiquement la partie supérieure du bras, le serrage étant assuré par une fermeture adhésive du type connu sous la dénomination commerciale « VELCRO », par exemple. La coquille 2 est, en outre, reliée à la coquille 3 par un tendeur 5, qui s'accroche sur la coquille 2 en un point de la zone postéro-supérieur de cette coquille, et sur la coquille 3 au droit de la partie sous-épineuse de l'omoplate, sensiblement dans la région centrale de l'omoplate. Le tendeur 5 passe sur une poulie 6, portée par la coquille 3 en un point situé au niveau de l'épine de l'omoplate, sensiblement au tiers à partir de l'extérieur.

La coquille 2 est également reliée à la coquille 1 par un tendeur 7, qui est accroché sur la coquille 2 en un point de la partie supérieure de cette coquille qui est au niveau de la gouttière du biceps. Le tendeur 7 est accroché sur la coquille 1 en un point situé au droit de la partie sus-épineuse interne de l'omoplate, dans la zone d'insertion du trapèze. Le tendeur 7 passe sur une poulie 8, portée par la coquille 1 et disposée au droit de la coracoïde.

La bande de caoutchouc, qui relie les coquilles 1 et 2, est disposée vers l'avant, du deltoïde moyen vers la clavicule. La bande de caoutchouc qui assure la liaison entre la coquille 2 et la coquille 3 est disposée vers l'arrière, du deltoïde moyen vers l'épine de l'omoplate. Les formes des coquilles 1, 2, 3 et les liaisons établies ménagent un angle de déplacement du bras par rapport à l'épaule d'environ 45° vers l'avant ou vers l'arrière. La bande de caoutchouc, qui assure la liaison entre les coquilles 1 et 3, est disposée sensiblement au-dessus de l'épine de l'omoplate.

Le deuxième sous-ensemble de la structure thoraco-scapulaire est le sous-ensemble d'amarrage. Ce sous-ensemble comporte une ceinture 9 entourant le sujet au niveau de la taille. A cette ceinture est relié une liaison antérieure constituée d'un levier-ressort thoracique 10 et d'un raccord élastique 11. Le levier-ressort thoracique 10 s'accroche sur la ceinture 9 au niveau de l'épine iliaque du côté opposé au membre appareillé ; il est lié au raccord élastique 11 au niveau de l'appendice xiphoïde, c'est-à-dire de la pointe du sternum. Le raccord élastique 11 est accroché sur la coquille 1 de l'épaulière en un point situé sur la partie antéro-inférieure de la coquille 1, au-dessous de la clavicule. Le raccord élastique 11 est constitué d'une bande élastique ; le levier-ressort thoracique 10 est constitué d'une lame métallique en acier de type « XC75 », la lame ayant une épaisseur de 0,5 mm et une largeur de 25 mm ; la longueur de la lame est fonction de l'anatomie du sujet. Il serait possible d'envisager de remplacer ce levier-ressort 10 par une transmission thoracique, qui serait uniquement constituée d'une courroie, mais le résultat semble moins bon dans ce cas quant à la transmission de l'énergie du moteur scapulaire sur le membre handicapé.

La ceinture 9 est raccordée par une première liaison élastique latérale 12 avec la coquille 3 de l'épaulière. La liaison élastique latérale est constituée d'une bande élastique ; elle s'accroche sur la ceinture 9 au niveau de l'épine iliaque du côté du membre appareillé ; elle s'accroche sur la coquille 3 au niveau de la pointe de l'omoplate.

Entre, d'une part, la zone de jonction du levier-ressort 10 avec le raccord élastique 11 et, d'autre part, la coquille 3, se trouve une deuxième liaison latérale 13, constituée d'une bande élastique. Le point d'attache de la bande 13 sur la coquille 3 est disposé dans la zone supérieure interne de la coquille 3, légèrement au-dessous de l'extrémité interne de l'épine de l'omoplate. Le positionnement de la bande 13 est tel que le mamelon du côté opposé au membre appareillé reste dégagé.

L'appareillage selon l'invention associe la structure thoraco-scapulaire ci-dessus décrite à un para-squelette du membre supérieur et de la main. Ce para-squelette comporte cinq éléments de liaison avec le membre handicapé. L'élément de liaison huméral supérieur est constitué de la coquille 2 et du lien périphérique 4 ; il s'étend sensiblement sur un tiers de la longueur du bras. L'élément de liaison huméral inférieur est constitué d'une coquille 14, qui présente du côté interne du bras une ouverture permettant l'introduction du membre. La coquille 14 est maintenue sur le membre au moyen d'un lien périphérique 15 et l'ensemble 14, 15 recouvre la partie humérale inférieure du bras, sur environ un tiers de la longueur du bras. Le lien périphérique 15 est constitué de deux bandes analogues à la bande 4, disposées aux extrémités supérieure et inférieure de la coquille 14.

L'élément de liaison de la partie radio-cubitale supérieure comporte, également, une coquille 16 analogue à la coquille 14. La coquille 16 est également ouverte du côté de la partie intérieure de l'avant-bras ; elle est fixée sur l'avant-bras par un lien périphérique 17, constitué de deux bandes élastiques analogues à la bande 4, les deux bandes étant placées aux extrémités supérieure et inférieure de la coquille 16. Les coquilles 14 et 16 sont séparées au niveau du coude par un espace 18, qui a une largeur sensiblement constante et égale à 8 mm. Pour permettre l'articulation de l'avant-bras par rapport au bras, la bordure inférieure de la coquille 14 présente deux formes convexes arrondies 14a au droit de l'épicondyle et de l'épitrochlée, ces deux formes convexes étant séparées par une concavité arrondie 14b au droit de l'olécrane. La bordure supérieure de la coquille 16 présente une forme complémentaire pour que l'espace articulaire 18 reste d'une largeur sensiblement constante. Cette découpe permet un mouvement relatif de la coquille 16 par rapport à la coquille 14, qui correspond à l'articulation du coude. Les coquilles 14 et 16 sont liées par deux tendeurs 19 et 20, disposés de part et d'autre de l'olécrane, au-dessus de l'espace articulaire 18. En outre, les coquilles 14 et 16 sont reliées entre elles par deux tendeurs élastiques 21 et 22, disposés respectivement dans le creux du coude, sur les bordures interne et externe du membre.

L'élément de liaison radio-cubital inférieur comporte une coquille constituée de deux demi-coquilles 23, 24 recouvrant respectivement la partie radiale et la partie cubitale de l'avant-bras. Ces deux demi-coquilles sont séparées par un sillon 25 et elles sont serrées autour du membre par deux liens périphériques 26, disposés aux deux extrémités supérieure et inférieure de ladite coquille 23, 24. L'élément de liaison radio-cubital inférieur s'étend sur sensiblement le tiers inférieur de l'avant-bras. Le membre est introduit entre les deux demi-coquilles 23, 24 par le côté opposé au sillon 25.

L'élément de liaison de la main comporte une coquille constituée de trois segments 27, 28, 29. Le segment 27 recouvre l'éminence thenar. Le segment 28 recouvre les deuxième, troisième et quatrième carpiens et métacarpiens jusqu'à la base des doigts. Le troisième segment 29 recouvre l'éminence hypothenar. Ces trois segments sont maintenus sur le calice de la main au moyen de deux liens périphériques. Un premier lien périphérique 30 est disposé sur les segments 28 et 29, au niveau de la partie inférieure des métacarpiens, et il entoure la main à l'exception du pouce. Un deuxième lien périphérique 31 entoure le carpe et passe sur les trois segments 27, 28, 29, au voisinage du poignet. Les liens périphériques 30 et 31 ont une largeur d'environ 2 cm. Le premier lien périphérique 30 est relié élastiquement à la zone cubitale inférieure de la demi-coquille 24 par un tendeur élastique 33 qui passe en diagonale au-dessus du carpe et du métacarpe et se fixe dans la partie palmaire du lien 30 sensiblement au droit de la base du majeur. Un tendeur élastique 32 est disposé entre la zone radiale inférieure de la demi-coquille 23 et le segment 27, au niveau de la base du pouce. L'élément de liaison de la main est relié à l'élément de liaison radio-cubital inférieur par une bande de caoutchouc disposée au-dessus du poignet.

Toutes les coquilles 14, 16, 23, 24, 27, 28, 29 du para-squelette sont réalisées en matière plastique moulée en fonction de l'anatomie du sujet. Tous les liens périphériques 15, 17, 26, 30, 31 sont analogues au lien 4.

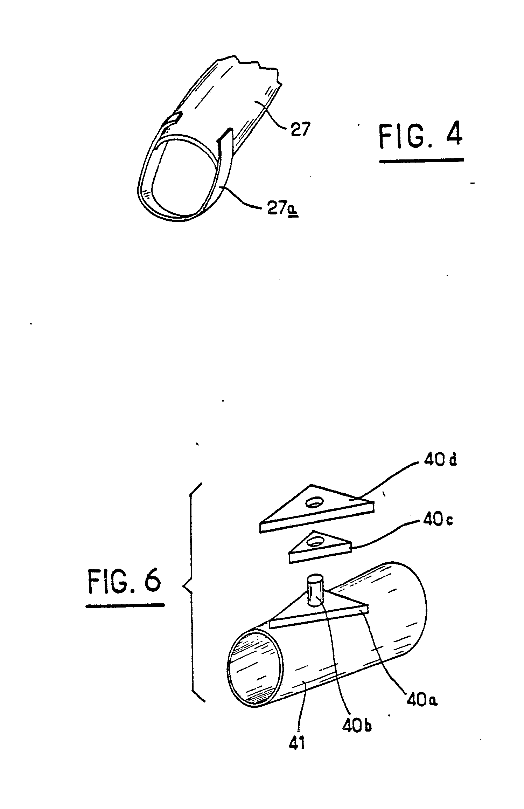

Dans la première variante représentée sur la figure 4, le segment 27 est équipé d'un lien 27a formant une anse et entourant la base de la colonne du pouce. Ce lien 27a peut être constitué d'une bande élastique insérée à l'extrémité inférieure du segment 27. Les autres doigts ne sont pas appareillés.

Dans une seconde variante, représentée sur les figures 5 et 6, chacun des doigts de la main est appareillé. Dans ce cas, chacun des segments 27, 28, 29 porte, au droit de chacun des métacarpiens, une borne d'appui désignée par 40 dans son ensemble. Le segment 27 porte donc une borne 40 ; le segment 28 porte trois bornes 40 ; et le segment 29 porte une borne 40. Toutes les bornes 40 sont identiques. Chaque borne 40 est constituée d'une ambase 40a de forme triangulaire supportant un axe cylindrique 40b sensiblement perpendiculaire à l'os qu'il surmonte. Sur l'embase 40a repose une came triangulaire 40c, qui est enfilée sur l'axe 40b et qui est maintenue en place contre l'embase 40a par un couvercle triangulaire 40d de même forme et de même dimension que l'embase 40a, le couvercle 40d étant également enfilé sur l'axe 40b. L'empilement 40a, 40c, 40d est maintenu serré, par exemple par encliquetage du couvercle 40d sur le sommet de l'axe 40b. La position de la came 40c entre l'embase 40a et le couvercle 40d est réglable en rotation autour de l'axe 40b, mais en raison du serrage de l'empilement, cette position est maintenue après réglage par friction de la came 40c entre l'embase 40a et le couvercle 40d. On peut prévoir que les surfaces en regard soient granitées ou striées pour assurer une bonne friction.

Chacun des doigts de la main est équipé au niveau de la deuxième phalange d'un doigtier 41 ayant une forme légèrement tronconique. Chaque doigtier 41 comporte une borne d'appui 40 identique à celle qui a été précédemment décrite. Toutes les bornes 40 sont disposées sur le dessus de la main. Pour chacun des doigts de la main, un tendeur élastique 42 est mis en place entre la borne d'appui 40 située au niveau de la deuxième phalange et la borne d'appui 40 située au niveau du métacarpe. Le tendeur 42 est croisé en X et passe autour de la came 40c de chacune des bornes 40 qui lui sont associées. De la sorte, en orientant convenablement la came 40c de chacune des deux bornes d'appui, on peut régler la direction de l'effort de compression appliqué sur les articulations du doigt. Les tendeurs 42 constituent des freins élastiques à la fermeture de la main.

Le para-squelette qui vient d'être décrit comporte, en outre, de façon essentielle pour le fonctionnement de l'appareillage selon l'invention, deux leviers-ressorts constitués de lames métalliques analogues aux leviers-ressorts 10. Le premier levier-ressort 34 est fixé entre un point de la zone centrale de la coquille 2 de l'épaulière et la coquille 14 ; il est sensiblement parallèle à l'humérus, le long de la bordure externe du bras. Le second levier-ressort 35 est fixé entre la coquille 16 et le segment 29 ; il est sensiblement parallèle au cubitus, en regard de l'espace compris entre le cubitus et le bord interne de l'avant-bras ; son accrochage sur le segment 29 s'effectue un peu en avant du pisiforme.

On constate que l'appareillage ci-dessus décrit permet à un handicapé moteur des membres supérieurs de retrouver une certaine mobilité de son membre handicapé.

En effet, les tensions des différents tendeurs des leviers-ressorts de l'apparillage sont réglées de façon qu'en position de repos l'avant-bras fasse environ un angle de 30° avec le bras et que le poignet soit en légère flexion dorsale (environ 10° vers le haut) ; en outre, les doigts sont en légère flexion palmaire (environ 15° vers le bas) et le pouce est en abduction de façon à ouvrir la pince de préhension.

Lorsque le sujet met son épaule en arrière, il ouvre le cône scapulaire constitué par l'omoplate et la clavicule, ce qui provoque la montée de l'humérus. Dans ce mouvement, le bras de levier de l'action du poids de l'avant-bras par rapport à l'articulation du coude augmente et il en résulte une légère extension par ouverture de l'angle du coude, les tendeurs 21, 22 freinant cette ouverture. Sous l'effet du poids, la main a tendance à descendre vers le bas, ce qui est freiné par les tendeurs 32, 33. La main a tendance à se fermer, ce qui est freiné par les tendeurs 42.

Si le sujet monte son épaule vers le haut et vers l'avant, on réalise un mouvement de retour par rapport au mouvement précédent. L'humérus est tiré en arrière ; le bras de levier du poids de l'avant-bras par rapport au coude diminue et sous l'action des tendeurs 21, 22 l'avant-bras remonte, ce qui ferme légèrement l'angle du coude. La main tourne légèrement en tendant à venir paume en l'air et les doigts s'ouvrent.

On constate donc que le moteur scapulaire permet, en combinant les différents mouvements possibles de l'épaule, la commande de l'appareillage selon l'invention. Les mouvements de l'humérus, quand le sujet déplace son épaule, sont non seulement commandés par les attaches physiologiques, mais également par le levier-ressort 34 qui, étant fixé sur la coquille 2 de l'épaulière, subit les efforts de traction appliqués sur l'épaulière par les moyens d'accrochage 9, 10, 11, 12, 13. Le mouvement est transmis du bras à la main par le levier-ressort 35. On neutralise ainsi d'une façon dynamique le poids du membre handicapé.

On a constaté que le travail du membre ainsi appareillé permet la réactivation des unités motrices, ce qui accélère la récupération du geste de préhension. En outre, le sujet qui utilise un tel appareillage et mobilise son membre handicapé recouvre d'une façon spectaculaire une grande partie de la sensibilité du membre.

Il est bien entendu que le mode de réalisation ci-dessus décrit n'est aucunement limitatif et pourra donner lieu à toutes modifications désirables, sans sortir pour cela du cadre de l'invention.

La présente description contient des éléments qui font l'objet d'une demande divisionnaire EP-A 0 226 068.

## Revendications

1. Appareillage externe pour handicapés moteurs d'au moins un membre supérieur, qui permet au sujet de mouvoir son membre handicapé, et qui, pour chaque membre handicapé, comporte :
une structure thoraco-scapulaire constituée d'une épaulière et d'un sous-ensemble d'amarrage (9, 10, 11, 12, 13) disposé autour du torse,
un para-squelette du membre supérieur et de la main, appareillage caractérisé en ce que :
1.1 l'épaulière est formée d'au moins trois coquilles semi-rigides (1, 2, 3) reliées entre elles de façon souple, la première (1) étant disposée sur l'angle supérieur du pectoral et passant sur l'arête supérieure du trapèze, la seconde (2) étant disposée sur la zone claviculo-trapézoïdale à la pointe de l'épaule, la troisième (3) étant disposée au droit du scapulum, au moins un tendeur élastique (5, 7) étant prévu entre la seconde coquille (2) et chacune des deux autres ;

1.2 le sous-ensemble d'amarrage (9, 10, 11, 12, 13) disposé autour du torse est relié à l'épaulière en au moins trois points d'accrochage répartis de part et d'autre de l'épaule ;

et que le para-squelette du membre supérieur et de la main comprend :

2.1 cinq éléments de liaison avec le membre disposés respectivement au niveau des parties humérales supérieure (2, 4) et inférieure (14, 15), des parties radio-cubitales supérieure (16, 17) et inférieure (23, 24, 26) et de la main (27, 28, 29, 30, 31), l'élément de liaison huméral supérieur comprenant la seconde coquille (2) de l'épaulière et un lien périphérique (4) qui entoure le membre, chacun des quatre autres éléments de liaison comportant une coquille semi-rigide (14, 16, 23, 24, 27, 28, 29) réalisée en une ou plusieurs pièces et au moins un lien périphérique (15, 17, 26, 30, 31) qui entoure le membre ;

2.2 des moyens élastiques disposés entre deux des éléments de liaison susmentionnés, qui comprennent :

2.2.1 un levier-ressort (34) fonctionnant en flexion et en torsion fixé entre la seconde coquille (2) de l'épaulière et la coquille (14) de l'élément de liaison huméral inférieur, sensiblement parallèlement à l'humérus le long de la bordure externe du membre,

2.2.2 un second levier-ressort (35) analogue au premier, fixé entre la coquille (16) de l'élément de liaison radio-cubital supérieur et la coquille de la main, sensiblement parallèlement au cubitus le long de la bordure interne du membre,

2.2.3 deux tendeurs élastiques (21, 22), disposés sur les bordures externe et interne du bras, entre les éléments de liaison huméral inférieur (14, 15) et radio-cubital supérieur (16, 17),

2.2.4 deux tendeurs élastiques (32, 33) disposés entre l'élément de liaison radio-cubital inférieur (23, 24, 26) et l'élément de liaison (27, 28, 29, 30, 31) de la main.

2. Appareillage selon la revendication 1, caractérisé par le fait que le sous-ensemble d'amarrage est relié à l'épaulière en trois points d'accrochage, le premier situé sur la zone antérieure de la première coquille (1), le second situé sur la zone inférieure de la troisième coquille (3), et le troisième situé sur la zone supérieure interne de la troisième coquille (3).

3. Appareillage selon la revendication 2, caractérisé par le fait que le sous-ensemble d'amarrage est constitué :

d'une ceinture (9) entourant le sujet au niveau de la taille ;

d'une liaison antérieure constituée d'une transmission thoracique (10) et d'un raccord élastique (11), la transmission (10) ayant une extrémité fixée sur la ceinture (9) au niveau de l'épine iliaque du côté opposé au membre appareillé et ayant son autre extrémité liée par le raccord élastique (11) précité à la première coquille (1) de l'épaulière ;

d'une première liaison élastique latérale (12) tendue entre la ceinture (9), au niveau de l'épine iliaque du côté du membre appareillé, et la troisième coquille (3) de l'épaulière ;

d'une deuxième liaison élastique latérale (13) tendue, du côté opposé au membre appareillé, entre la troisième coquille (3) de l'épaulière et la liaison antérieure (10, 11) précitée.

4. Appareillage selon la revendication 3, caractérisé par le fait que la transmission thoracique est un levier-ressort (10), qui forme un angle d'environ 30° avec l'axe du corps du sujet ; que la première liaison élastique latérale (12) se raccorde à la troisième coquille (3) de l'épaulière au droit de la base de l'omoplate ; et que la deuxième liaison élastique latérale (13) se raccorde à la troisième coquille (3) de l'épaulière, au voisinage de la partie interne de l'épine de l'omoplate, et à la liaison antérieure (10, 11) au voisinage de l'appendice xiphoïde.

5. Appareillage selon l'une des revendications 1 à 4, caractérisé par le fait que la seconde coquille (2) de l'épaulière est reliée à la troisième coquille (3) par un tendeur (5) fixé entre la zone postéro-supérieure de la seconde coquille (2) et la zone centrale de la troisième coquille (3), un renvoi d'angle (6) obligeant le tendeur (5) à passer au droit de la partie externe de l'épine de l'omoplate.

6. Appareillage selon l'une des revendications 1 à 5, caractérisé par le fait que la seconde coquille (2) de l'épaulière est reliée à la première coquille (1) par un tendeur (7), fixé entre la zone antéro-supérieure de la deuxième coquille (2) et la zone postéro-interne de la première coquille (1), un renvoi d'angle (8) obligeant le tendeur (7) à rester au droit de la zone claviculaire sur environ la moitié de sa longueur.

7. Appareillage selon l'une des revendications 5 ou 6, caractérisé par le fait que les renvois d'angles des tendeurs (5, 7) sont constitués par des poulies (6, 8).

8. Appareillage selon l'une des revendications 1 à 7, caractérisé par le fait que le leviers-ressorts sont constitués par des lames métalliques flexibles.

9. Appareillage selon l'une des revendications 1 à 8, caractérisé par le fait que l'élément de liaison huméral inférieur (14, 15) et l'élément de liaison radio-cubital supérieur (16, 17) comportent chacun une seule coquille, les deux coquilles (14, 16) ayant leurs bordures adjacentes séparées par un espace articulaire (18) ayant une largeur sensiblement constante et étant reliées au droit dudit espace (18) par des moyens élastiques (19, 20) disposés de part et d'autre de l'olécrane, la bordure de la coquille humérale inférieure (14) le long dudit espace (18) présentant une forme convexe (14a) au droit de l'épicondyle et de l'épitrochlée et une forme concave (14b) au droit de l'olécrane, la bordure de la coquille radio-cubitale supérieure (16) présentant la forme complémentaire.

10. Appareillage selon l'une des revendications 1 à 9, caractérisé par le fait que la coquille de l'élément de liaison radio-cubital inférieur est constituée de deux demi-coquilles (23, 24) recouvrant respectivement la partie radiale et la partie cubitale du membre, ces deux demi-coquilles étant séparées par un sillon (25) au droit du cubitus, le membre étant introduit dans la coquille par le côté opposé au sillon (25), les deux demi-coquilles (23, 24) étant serrées autour du membre par au moins un lien périphérique (26).

11. Appareillage selon l'une des revendications 1 à 10, caractérisé par le fait que l'élément de liaison radio-cubital inférieur (23, 24, 26) comporte une coquille (23, 24), qui est serrée autour du membre par deux liens périphériques (26) disposés aux deux extrémités supérieure et inférieure de ladite coquille.

12. Appareillage selon l'une des revendications 1 à 11, caractérisé par le fait que l'élément de liaison huméral inférieur (14, 15) ou supérieur (2, 4) recouvre environ le tiers du bras et que l'élément de liaison radio-cubital inférieur (23, 24, 26) ou supérieur (16, 17) recouvre environ le tiers de l'avant-bras.

13. Appareillage selon l'une des revendications 1 à 10, caractérisé par le fait que l'élément de liaison de la main comporte, en premier lieu, une coquille constituée de trois segments, le premier segment (27) recouvrant l'éminence thenar, le deuxième segment (28) recouvrant les deuxième, troisième et quatrième carpiens et métacarpiens jusqu'à la base des doigts, le troisième segment (29) recouvrant l'éminence hypothenar, et, en second lieu, d'une part, un premier lien périphérique (30) disposé sur les premier et deuxième segments (27, 28) et entourant la main à l'exception du pouce au niveau de la partie inférieure des métacarpiens, et, d'autre part, un deuxième lien périphérique (31) entourant le carpe et passant sur les trois segments (27, 28, 29) au voisinage du poignet, le deuxième lien périphérique (31) étant relié à l'élément de liaison radio-cubital inférieur (23, 24, 26) par une bande de caoutchouc (43).

14. Appareillage selon la revendication 13, caractérisé par le fait que l'élément de liaison de la main (27, 28, 29, 30, 31) est relié à l'élément de liaison radio-cubital inférieur (23, 24, 26) par deux tendeurs (32, 33), l'un (32) entre la base du pouce et la zone radiale inférieure, l'autre (33) entre la zone cubitale inférieure et la zone palmaire médiane du métacarpe, passant en diagonale au-dessus de la main.

15. Appareillage selon l'une des revendications 13 ou 14, caractérisé par le fait que le second levier-ressort (35) est disposé en regard de l'espace entre le cubitus et le bord interne de l'avant-bras et qu'il a son extrémité inférieure fixée sur le troisième segment (29) de la coquille de la main, un peu en avant du pisiforme.

16. Appareillage selon l'une des revendications 13 à 15, caractérisé par le fait que le premier segment (27) de la coquille de l'élément de liaison de la main porte un lien (27a), qui entoure la base du pouce.

17. Appareillage selon l'une des revendications 13 à 15, caractérisé par le fait que chaque doigt de la main est appareillé par un tendeur élastique croisé (42), disposé entre une borne d'appui (40) placée au-dessus du métacarpe et une borne d'appui (40) placée au-dessus de la seconde phalange.

18. Appareillage selon la revendication 17, caractérisé par le fait que la borne d'appui (40) placée au niveau du métacarpe est portée par le segment de la coquille de la main, qui lui correspond, et que la borne d'appui (40) placée au niveau de la seconde phalange est portée par un doigtier (41), qui entoure l'extrémité du doigt.

19. Appareillage selon l'une des revendications 17 ou 18, caractérisé par le fait que chaque borne d'appui (40) comporte un élément triangulaire (40c) à orientation réglable susceptible de tourner autour d'un axe (40b) sensiblement perpendiculaire à l'os qu'il surmonte.

**Claims**

1. External fitting for persons with a motor handicap in at least one upper limb, which enables the patient to move his handicapped limb and which, for each handicapped limb, comprises :

a thoraco-scapular structure consisting of a shoulder strap and of a fastening subassembly (9, 10, 11, 12, 13) arranged round the torso, and

a para-skeleton of the upper limb and of the hand, the said fitting being characterized in that :

1.1 the shoulder strap is formed from at least three semi-rigid shells (1, 2, 3) connected flexibly to one another, the first (1) being arranged on the upper corner of the pectoral and passing over the upper edge of the trapezius, the second (2) being arranged on the claviculotrapezoidal zone at the tip of the shoulder, and the third (3) being arranged in line with the scapulum, at least one elastic tensioner (5, 7) being provided between the second shell (2) and each of the other two ;

1.2 the fastening subassembly (9, 10, 11, 12, 13) arranged round the torso is connected to the shoulder strap at at least three attachment points distributed on either side of the shoulder ;

and in that the para-skeleton of the upper limb and of the hand comprises :

2.1 five elements for connection to the limb, which are arranged respectively in the region of the upper and lower humeral parts (2, 4 and 14, 15), of the upper and lower cubitoradial parts (16, 17 and 23, 24, 26) and of the hand (27, 28, 29, 30, 31), the upper humeral connection element comprising the second shell (2) of the shoulder strap and a peripheral tie (4) surrounding the limb, each of the other four connection elements comprising a semi-rigid shell (14, 16, 23, 24, 27, 28, 29) produced in one or more parts and at least one peripheral tie (15, 17, 26, 30, 31) surrounding the limb ;

2.2 elastic means arranged between two of the abovementioned connection elements and comprising :

2.2.1 a spring lever (34) functioning in flexure and torsion and secured between the second shell (2) of the shoulder strap and the shell (14) of the lower humeral connection element, substantially parallel to the humerus and along the outer margin of the limb,

2.2.2 a second spring lever (35) similar to the first and secured between the shell (16) of the upper cubitoradial connection element and the shell of the hand, substantially parallel to the cubitus and along the inner margin of the limb,

2.2.3 two elastic tensioners (21, 22) arranged on the outer and inner margins of the arm between the lower humeral connection element (14, 15) and the upper cubitoradial connection element (16, 17),

2.2.4 two elastic tensioners (32, 33) arranged between the lower cubitoradial connection element (23, 24, 26) and the connection element (27, 28, 29, 30, 31) of the hand.

2. Fitting according to Claim 1, characterized in that the fastning subassembly is connected to the shoulder strap at three attachment points, the first located on the front zone of the first shell (1), the second located on the lower zone of the third shell (3) and the third located on the inner upper zone of the third shell (3).

3. Fitting according to Claim 2, characterized in that the fastening subassembly consists of :

a belt (9) surrounding the patient at the waist ;

a front connection consisting of a thoracic transmission (10) and of an elastic link (11), the transmission (10) having one end secured to the belt (9) in the region of the spine of the hip on the opposite side to the fitted limb and having its other end connected to the first shell (1) of the shoulder strap by means of the abovementioned elastic link (11) ;

a first lateral elastic connection (12) stretched between the belt (9), in the region of the spine of the hip on the same side as the fitted limb, and the third shell (3) of the shoulder strap ;

a second lateral elastic connection (13) stretched, on the opposite side to the fitted limb, between the third shell (3) of the shoulder strap and the abovementioned front connection (10, 11).

4. Fitting according to Claim 3, characterized in that the thoracic transmission is a spring lever (10) which forms an angle of approximately 30° with the axis of the patient's body, in that the first lateral elastic connection (12) is joined to the third shell (3) of the shoulder strap in line with the base of the shoulder blade, and in that the second lateral elastic connection (13) is joined to the third shell (3) of the shoulder strap near the inner part of the spine of the shoulder blade and to the front connection (10, 11) near the xiphoid appendage.

5. Fitting according to one of Claims 1-4, characterized in that the second shell (2) of the shoulder strap is connected to the third shell (3) by means of a tensioner (5) secured between the upper rear zone of the second shell (2) and the central zone of the third shell (3), a guide device (6) forcing the tensioner (5) to pass in line with the outer part of the spine of the shoulder blade.

6. Fitting according to one of Claims 1-5, characterized in that the second shell (2) of the shoulder strap is connected to the first shell (1) by means of a tensioner (7) secured between the upper front zone of the second shell (2) and the inner rear zone of the first shell (1), a guide device (8) forcing the tensioner (7) to remain in line with the clavicular zone over approximately half its length.

7. Fitting according to one of Claims 5 or 6, characterized in that the guide devices of the tensioners (5, 7) consist of pulleys (6, 8).

8. Fitting according to one of Claims 1-7, characterized in that the spring levers consist of flexible metal blades.

9. Fitting according to one of Claims 1-8, characterized in that the lower humeral connection element (14, 15) and the upper cubitoradial connection element (16, 17) each comprise a single shell, the two shells (14, 16) having their adjacent edges separated by an articular space (18) of substantially constant width and being connected in line with the said space (18) by elastic means (19, 20) arranged on either side of the olecranon, the edge of the lower humeral shell (14) along the said space (18) having a convex form (14a) in line with the epicondyle and the epitrochlea and a concave form (14b) in line with the olecranon, the edge of the upper cubitoradial shell (16) having a matching form.

10. Fitting according to one of Claims 1-9, characterized in that the shell of the lower cubitoradial connection element consists of two half-shells (23, 24) respectively covering the radial part and the cubital part of the limb, these two half-shells being separated by means of a groove (25) in line with the cubitus, the limb being introduced into the shell from the side opposite the groove (25), the two half-shells (23, 24) being clamped round the limb by means of at least one peripheral tie (26).

11. Fitting according to one of Claims 1-10, characterized in that the lower cubitoradial connection element (23, 24, 26) comprises a shell (23, 24) which is clamped round the limb by means of two peripheral ties (26) arranged at the two upper and lower ends of the said shell.

12. Fitting according to one of Claims 1-11, characterized in that the lower humeral connection element (14, 15) or upper humeral connection element (2, 4) covers approximately one third of the arm, and in that the lower cubitoradial connection element (23, 24, 26) or upper cubitoradial connection element (16, 17) covers approximately one third of the forearm.

13. Fitting according to one of Claims 1-10, characterized in that the connection element for the hand comprises, in the first place, a shell consisting of three segments, the first segment (27) covering the ball of the thumb, the second segment (28) covering the second, third and fourth carpals and metacarpals up to the base of the fingers, and the third segment (29) covering the hypothenar, and, in the second place, on the one hand the first peripheral tie (30) arranged on the first and second segments (27, 28) and surrounding the hand, with the exception of the thumb, in the region of the lower part of the metacarpals, and on the other hand a second peripheral tie (31) surrounding the carpal and passing over the three segments (27, 28, 29) in the region of the wrist, the second peripheral tie (31) being connected to the lower cubitoradial connection element (23, 24, 26) by means of a rubber band (43).

14. Fitting according to Claim 13, characterized in that the connection element for the hand (27, 28, 29, 30, 31) is connected to the lower cubitoradial connection element (23, 24, 26) by means of two tensioners (32, 33), one (32) between the base of the thumb and the lower radial zone and the other (33) between the lower cubital zone and the middle palmar zone of the metacarpal, passing diagonally over the hand.

15. Fitting according to one of Claims 13 and 14, characterized in that the second spring lever (35) is arranged opposite the space between the cubitus and the inner edge of the forearm, and in that its lower end is secured to the third segment (29) of the shell of the hand a little in front of the pisiform bone.

16. Fitting according to one of Claims 13 to 15, characterized in that the first segment (27) of the shell of the connection element for the hand carries a tie (27a) which surrounds the base of the thumb.

17. Fitting according to one of Claims 13 to 15, characterized in that each finger of the hand is fitted with a crossed elastic tensioner (42) arranged between a bearing post (40) located above the metacarpal and a bearing post (40) located above the second phalanx.

18. Fitting according to Claim 17, characterized in that the bearing post (40) located in the region of the metacarpal is carried by the segment of the hand shell corresponding to it, and in that the bearing post (40) located in the region of the second phalanx is carried by a fingerstall (41) surrounding the end of the finger.

19. Fitting according to one of Claims 17 or 18, characterized in that each bearing post (40) has a triangular element (40c) of adjustable orientation, capable of rotating about a pivot (40b) substantially perpendicular to the bone above which it is placed.

**Patentansprüche**

1. Äußere Vorrichtung für motorisch Behinderte an zumindest einer der oberen Extremitäten, die der Person die Bewegung ihrer behinderten Extremität ermöglicht und für jede behinderte Extremität folgende Teile umfaßt :
einen Thorax-Schultergürtel-Aufbau bestehend aus einem Schulterstück und einem um den Rumpf liegenden Befestigungsbauteil (9, 10, 11, 12, 13),
ein Nebenskelett der oberen Extremität und der Hand, dadurch gekennzeichnet, daß :
1.1 das Schulterstück zumindest aus drei halbstarren Schalen (1, 2, 3) besteht, die flexibel miteinander verbunden sind, wobei die erste (1) am oberen Winkel des Brustmuskels zu liegen kommt und über den oberen Rand des Trapezmuskels verläuft, die zweite (2) im Bereich des Schlüsselbeins und Trapezmuskels an der Schulterspitze angeordnet ist und die dritte (3) senkrecht zum Schulterblatt liegt, wobei zumindest ein elastischer Spanner (5, 7) zwischen der zweiten (2) und zwischen jeder der beiden anderen Schalen vorgesehen ist ;
1.2 der um den Rumpf liegende Befestigungsbauteil (9, 10, 11, 12, 13) mit dem Schulterstück an zumindest drei zu beiden Seiten der Schulter verteilten Aufhängepunkten verbunden ist ;
und daß das Nebenskelett der oberen Extremität und der Hand folgende Teile umfaßt :
2.1 fünf, der Verbindung mit der Extremität dienende Elemente, die an den oberen (2, 4) bzw. unteren Humerusanteilen (14, 15), den oberen (6, 17) und unteren (23, 24, 26) radioulnaren Anteilen und der Hand (27, 28, 29, 30, 31) angeordnet sind, wobei das obere humerale Verbindungselement die zweite Schale (2) des Schulterstücks und ein die Extremität umgebendes peripheres Band (4) umfaßt, wobei jedes der vier anderen Verbindungselemente eine halbstarre, aus einem Teil oder aus mehreren Teilen hergestellte Schale (14, 16, 23, 24, 27, 28, 29) und zumindest ein die Extremität umgebendes peripheres Band (15, 17, 26, 30, 31), aufweist ;
2.2 elastische, zwischen zwei der oben angeführten Verbindungselemente angeordnete Mittel, die folgende Teile umfassen :
2.2.1 einen bei Beugung und Drehung arbeitenden, zwischen der zweiten Schale (2) des Schulterstücks und der Schale (14) des unteren humeralen Verbindungselements befestigten Federhebel (34), der längs des äußeren Randes der Extremität annähernd parallel zum Humerus liegt,
2.2.2 einen zweiten, dem ersten ähnlichen Federhebel (35), der zwischen der Schale (16) des oberen radioulnaren Verbindungselements und der Handschale befestigt ist, annähernd parallel zur Ulna längs des inneren Randes der Extremität,
2.2.3 zwei am äußeren und inneren Rand des Arms, zwischen den unteren humeralen (14, 15) und oberen radioulnaren (16, 17) Verbindungselementen angeordnete elastische Spanner (21, 22),
2.2.4 zwei zwischen dem unteren radioulnaren (23, 24, 26) Verbindungselement und dem Hand-Verbindungselement (27, 28, 29, 30, 31) angeordnete elastische Spanner.

2. Vorrichtung nach Anspruch 1, dadurch gekennzeichnet, daß der Befestigungsbauteil mit dem Schulterstück über drei Aufhängepunkte verbunden ist, wobei der erste im vorderen Bereich der ersten Schale (1), der zweite im unteren Bereich der dritten Schale (3) und der dritte im oberen, inneren Bereich der dritten Schale (3) liegt.

3. Vorrichtung nach Anspruch 2, dadurch gekennzeichnet, daß der Befestigungsbauteil besteht aus :
einem Gürtel (9), der die Person in der Taille umgibt ;

einer vorderen Verbindung, die aus einer thorakalen Transmission (10) und einem elastischen Verbindungsstück (11) besteht, wobei ein Ende der Transmission (10) auf dem Gürtel (9) in Höhe der der versorgten Extremität gegenüberliegenden Spina iliaca befestigt und das andere Ende mit der ersten Schale (1) des Schulterstücks über das oben angeführte elastische Verbindungsstück (11) verbunden ist,

einer ersten seitlichen elastischen Verbindung (12), die zwischen Gürtel (9) in Höhe der Spina iliaca auf der Seite der versorgten Extremität und der dritten Schale (3) des Schulterstücks verläuft,

einer zweiten seitlichen elastischen Verbindung (13), die an der der versorgten Extremität gegenüberliegenden Seite zwischen der dritten Schale (3) des Schulterstücks und der oben erwähnten vorderen Verbindung (10, 11) verläuft.

4. Vorrichtung nach Anspruch 3, dadurch gekennzeichnet, daß die thorakale Transmission ein Federhebel (10) ist, der mit der Körperachse der Person einen Winkel von etwa 30° bildet ; daß die erste seitliche elastische Verbindung (12) senkrecht zur Schulterblattbasis verlaufend mit der dritten Schale (3) des Schulterstücks verbunden ist, und die zweite seitliche elastische Verbindung (13) nahe dem inneren Teil der Spina scapulae verlaufend mit der dritten Schale (3) des Schulterstücks und nahe dem Processus xyphoideus verlaufend mit der vorderen Verbindung (10, 11) verbunden ist.

5. Vorrichtung nach einem der Ansprüche 1 bis 4, dadurch gekennzeichnet, daß die zweite Schale (2) des Schulterstücks mit der dritten Schale (3) über einen Spanner (5) verbunden ist, der zwischen dem hinteren oberen Bereich der zweiten Schale (2) und dem zentralen Bereich der dritten Schale (3) befestigt ist, wobei ein Umlenkwinkelstück (6) den Spanner (5) senkrecht zum äußeren Teil der Spina scapulae verlagert.

6. Vorrichtung nach einem der Ansprüche 1 bis 5, dadurch gekennzeichnet, daß die zweite Schale (2) des Schulterstücks mit der ersten Schale (1) über einen Spanner (7) verbunden ist, der zwischen dem vorderen oberen Bereich der zweiten Schale (2) und dem hinteren inneren Bereich der ersten Schale (1) befestigt wird, wobei ein Umlenkwinkelstück (8) den Spanner (7) auf etwa der Hälfte seiner Länge senkrecht zum klavikularen Bereich hält.

7. Vorrichtung nach einem der Ansprüche 5 oder 6, dadurch gekennzeichnet, daß die Spanner-Umlenkwinkelstücke (5, 7) aus Rollen (6, 8) bestehen.

8. Vorrichtung nach einem der Ansprüche 1 bis 7, dadurch gekennzeichnet, daß die Federhebel aus flexiblen Metallplättchen bestehen.

9. Vorrichtung nach einem der Ansprüche 1 bis 8, dadurch gekennzeichnet, daß das untere humerale Verbindungselement (14, 15) und das obere radioulnare Verbindungselement (16, 17) jeweils eine einzige Schale aufweisen, wobei die aneinandergrenzenden Ränder beider Schalen (14, 16) durch einen Gelenksraum (18) annähernd konstanter Breite voneinander beabstandet und quer zu diesem Raum (18) geradlinig durch beiderseits vom Olekranum angeordnete Mittel (19, 20) miteinander verbunden sind, wobei der längs dieses Raumes (18) verlaufende Rand der unteren Humerusschale (14) eine geradlinig zum Epicondylus lateralis humeri und Epicondylus medialis humeri senkrechte, konvexe Form (14a) und eine zum Olekranum senkrechte, konkave Form (14b) aufweist, wobei der Rand der oberen radioulnaren Schale (16) die Komplementärform aufweist.

10. Vorrichtung nach einem der Ansprüche 1 bis 9, dadurch gekennzeichnet, daß die Schale des unteren radioulnaren Verbindungselements aus zwei Halbschalen (23, 24) besteht, welche den radialen bzw. ulnaren Teil der Extremität bedecken, wobei diese beiden Halbschalen durch eine zur Ulna senkrecht liegende Rille (25) voneinander getrennt sind, wobei die Extremität von der der Rille (25) gegenüberliegenden Seite in die Schale eingeführt wird, wobei die beiden Halbschalen (23, 24) durch zumindest ein peripheres Band (26) um die Extremität gespannt werden.

11. Vorrichtung nach einem der Ansprüche 1 bis 10, dadurch gekennzeichnet, daß das untere radioulnare Verbindungselement (23, 24, 26) eine Schale umfaßt (23, 24), die durch zwei periphere Bänder (26), welche am oberen und am unteren Ende der genannten Schale angeordnet sind, um die Extremität gespannt wird.

12. Vorrichtung nach einem der Ansprüche 1 bis 11, dadurch gekennzeichnet, daß das untere (14, 15) oder obere (2, 4) humerale Verbindungselement ungefähr ein Drittel des Armes bedeckt, und daß das untere (23, 24, 26) oder obere (16, 17) radioulnare Verbindungselement ungefähr ein Drittel des Vorderarms bedeckt.

13. Vorrichtung nach einem der Ansprüche 1 bis 10, dadurch gekennzeichnet, daß das Hand-Verbindungselement in erster Linie eine aus drei Segmenten gebildete Schale umfaßt, wobei das erste Segment (27) den Daumenballen, das zweite Segment (28) den zweiten, dritten und vierten Handwurzel- und Mittelhandknochen bis zur Fingerbasis und das dritte Segment den Kleinfingerballen bedeckt und in zweiter Linie einerseits ein an den ersten und zweiten Segmenten (27, 28) anliegendes peripheres Band (30), das die Hand mit Ausnahme des Daumens in der Höhe des unteren Teiles der Mittelhand umschließt sowie andererseits ein zweites peripheres Band (31), das die Handwurzel umgibt und über die drei Segmente (27, 28, 29) nahe dem Handgelenk verläuft, wobei das zweite periphere Band (31) mit dem unteren radioulnaren Verbindungselement (23, 24, 26) durch ein Gummiband verbunden ist.

14. Vorrichtung nach Anspruch 13, dadurch gekennzeichnet, daß das Hand-Verbindungselement (27, 28, 29, 30, 31) mit dem unteren radioulnaren Verbindungselement (23, 24, 26) durch zwei Spanner (32, 33) verbunden ist, wobei der eine (32) zwischen der Daumenbasis und dem unteren Radialbereich und der andere (33) zwischen dem unteren Ulnabereich und dem mittleren palmaren Bereich des Metacarpus diagonal über der Hand angeordnet ist.

15. Vorrichtung nach einem der Ansprüche 13 oder 14, dadurch gekennzeichnet, daß der zweite Federhebel (35) gegenüber dem Raum zwischen Ulna und internem Rand des Vorderarms angeordnet und sein unteres Ende auf dem dritten Segment (29) der Handschale geringfügig vor dem os pisiforme befestigt ist.

16. Vorrichtung nach einem der Ansprüche 13 bis 15, dadurch gekennzeichnet, daß das erste Schalensegment (27) des Hand-Verbindungsstücks ein Band (27a) aufweist, das die Daumenbasis umschließt.

17. Vorrichtung nach einem der Ansprüche 13 bis 15, dadurch gekennzeichnet, daß jeder Finger der Hand mit einem gekreuzten elastischen Spanner (42) versorgt wird, der zwischen einer oberhalb des Metacarpus liegenden Stützklemme (40) und einer oberhalb der zweiten Phalanx liegenden Stützklemme (40) zu liegen kommt.

18. Vorrichtung nach Anspruch 17, dadurch gekennzeichnet, daß die in der Höhe des Metacarpus liegende Stützklemme (40) vom entsprechenden Segment der Handschale getragen wird, und daß sich die in der Höhe der zweiten Phalanx befindliche Stützklemme (40) von einem das Fingerende umschließenden Fingerling (41) getragen wird.

19. Vorrichtung nach einem der Ansprüche 17 oder 18, dadurch gekennzeichnet, daß jede Stützklemme (40) ein einstellbares dreieckförmiges Element (40c) aufweist, das um eine zum Knochen annähernd senkrecht stehende Achse (40b) drehbar ist.

FIG. 1

FIG. 2

FIG. 3

FIG. 5

0 143 132

FIG. 4

FIG. 6